**Europäisches Patentamt**

**European Patent Office**

**Office européen des brevets**

(19)

(11) Numéro de publication : **0 014 130**
**B1**

(12) **FASCICULE DE BREVET EUROPÉEN**

(45) Date de publication du fascicule du brevet :
07.09.88

(51) Int. Cl.⁴ : **A 61 M 1/12, A 61 F 2/22**

(21) Numéro de dépôt : 80400067.7

(22) Date de dépôt : 16.01.80

(54) Prothèse cardiaque totale et dispositif de régulation de son débit sanguin.

(30) Priorité : 22.01.79 FR 7901529
29.11.79 FR 7929365

(43) Date de publication de la demande :
06.08.80 Bulletin 80/16

(45) Mention de la délivrance du brevet :
07.09.88 Bulletin 88/36

(84) Etats contractants désignés :
DE GB IT

(56) Documents cités :
FR-A- 2 215 930
FR-A- 2 278 349
US-A- 3 774 243
US-A- 4 051 841
SCIENTIFIC AMERICAN, vol. 213, no. 5, novembre 1965, pages 38-46 New York, U.S.A. W.J. KOLFF: "An Artificial Heart inside the Body"
MEDICAL AND BIOLOGICAL ENGINEERING, vol. 13, no. 5, septembre 1975 pages 662-668 New York, U.S.A. F. KLIMES et al.: "Analysis and model of controlling system to control heart rate and stroke volumes of an artificial heart"
INTERNATIONAL CONFERENCE ON BIOMEDICAL TRANSDUCERS, novembre 1975 pages 235-247 Paris, FR. K.M. RAGULSKIS et al.: "Capteurs de pression pour coeur artificiel"
J.J. SCHIPPERHEYN: Influence de la pression intra-ventriculaire sur la répartition de tension intramurale dans la préparation d'un coeur-poumon isolé du chien", pages 235-247

(73) Titulaire : **HELEN INDUSTRIAL PROPERTIES B.V.**
Locatellikade 1
NL-1076 AZ Amsterdam (NL)

(72) Inventeur : **Lapeyre, Didier,Dr.**
**Chaignes**
**F-27120 Pacy sur Eure (FR)**

(74) Mandataire : **Ores, Irène et al**
**CABINET ORES 6, Avenue de Messine**
**F-75008 Paris (FR)**

L'ONDE ELECTRIQUE, vol. 57, no. 2, février 1977, pages 142-147 Lyon, FR. R.C. BLANCHET: "Possibilités d'un organe moteur à commande électronique pour coeur artificiel"

BIO-MEDICAL ENGINEERING, vol. 12, no. 3 et no. 4, 7 octobre 1965, pages 173-186 M.G. CHESNUT et al.: "Assisted Circulation Controlled by Electronic Computation"

## Description

La présente invention est relative à une nouvelle prothèse cardiaque totale.

Comme on le sait, une cause importante de décès chez l'être humain est représentée par l'attaque cardiaque coronarienne : l'occlusion des artères coronaires provoque l'ischémie du myocarde, puis, si le débit sanguin est insuffisant, l'infarctus (ou nécrose) du myocarde. L'infarctus peut alors provoquer un état de choc cardiogénique qui nécessite, pour tenter d'assurer la survie du patient, la mise en œuvre de moyens d'assistance circulatoire d'urgence. Toutefois ces dispositifs d'assistance circulatoire impliquent pour le patient une très grande dépendance, attendu qu'il s'agit de le relier en permanence à des pompes extra-corporelles, l'interruption de sa dépendance étant généralement de nature à entraîner le décès. Différentes solutions à ce grave problème ont été envisagées, au nombre desquelles il faut citer la transplantation cardiaque, l'implantation d'une prothèse cardiaque partielle constituée par un ventricule gauche auxiliaire et la prothèse cardiaque artificielle totale.

On connaît les problèmes que pose la transplantation cardiaque : rareté des donneurs, histocompatibilité, rejet des greffes et insuffisance des réponses apportées par l'immunologie à ce rejet. Ce sont ces problèmes, qui n'ont pas reçu à ce jour de solutions satisfaisantes, qui ont conduit les Chercheurs à envisager de substituer à un cœur incapable d'exercer sa fonction, un organe artificiel qui n'entraînerait pas les réactions de rejet immunologique liées aux greffes cardiaques. Les recherches se sont donc tout d'abord orientées vers une prothèse auxiliaire de ventricule gauche qui serait implantée en association avec le cœur du patient ; si une telle prothèse peut être considérée comme valable pour remédier à la défaillance du ventricule gauche qui est l'origine la plus fréquente des défaillances cardiaques, elle ne serait cependant pas en mesure de remédier aux troubles possibles des parties subsistantes du cœur malade du patient, puisque celles-ci demeureraient en place. La solution théoriquement idéale serait donc celle représentée par le remplacement du cœur défaillant par une prothèse cardiaque totale. Un certain nombre d'équipes de Chercheurs dans le Monde, aux Etats-Unis, en République Fédérale d'Allemagne, au Japon, en Italie, notamment, expérimentent actuellement des prothèses cardiaques sur des animaux. Ces prothèses cardiaques sont des prothèses doubles du type « pompe à membranes » ; elles comportent un ventricule droit et un ventricule gauche activés chacun de façon indépendante par deux sources d'énergie séparées. Les prothèses cardiaques étudiées actuellement sont conçues pour se conformer au principe de STARLING selon lequel les variations du débit sanguin sollicitées par le réseau circulatoire du porteur de la prothèse sont obtenues par les variations du volume de sang admis dans chaque ventricule sous l'effet des variations de la pression de remplissage, tandis que la fréquence d'activation de la prothèse est maintenue constante. Les prothèses cardiaques proposées conformément à l'Art antérieur sont constituées par un ventricule droit et un ventricule gauche indépendants l'un de l'autre, pour faciliter la fabrication et l'implantation de la prothèse. L'indépendance des deux ventricules détermine l'existence entre eux d'un espace mort anatomique relativement important qui réduit le volume physiologiquement utilisable et constitue une prothèse dont l'encombrement est excessif.

De plus, la réalisation de ces prothèses sous forme de deux ventricules juxtaposés indépendants a pour effet de nécessiter l'activation des deux ventricules indépendamment l'un de l'autre à partir de sources d'énergie indépendantes, ce qui requiert une régulation entre les débits pompés par chacune des deux prothèses indépendantes. D'autre part, la conception des prothèses cardiaques connues, qui fait dépendre les variations du débit, des variations du volume systolique dues aux variations de la pression de remplissage, nécessite un grand volume de réserve pour répondre aux variations des besoins de l'organisme ; ainsi, une telle prothèse, réalisée avec des volumes ventriculaires correspondants aux exigences physiologiques, peut difficilement respecter, en même temps, les exigences d'encombrement déterminées par les dimensions de la cavité péricardique. En outre, en raison de leur volume et de leur encombrement relativement importants, ces doubles prothèses cardiaques font, dans une certaine mesure, obstacle à la circulation de retour. Dans la mesure où l'on réduit le volume et l'encombrement de telles prothèses, on obtient des performances insuffisantes et, par suite, un débit sanguin insuffisant lorsque les besoins de l'organisme augmentent (exercice musculaire par exemple). Le brevet français 2 278 349, déposé au nom de GOVERNMENT OF THE UNITED STATES, SECRETARY OF THE DEPARTMENT OF HEALTH, EDUCATION AND WELFARE, a trait à une prothèse cardiaque totalement implantable, qui est monobloc, dont les moyens d'activation sont placés dans la prothèse, entre les ventricules droit et gauche, lesdits moyens commandant par un système de came et de transmission mécanique le déplacement de deux plaques d'appui sur des membranes souples limitant lesdits ventricules, le retour des plaques à leur position initiale étant assuré par des moyens de ressort.

En outre, l'assemblage de la prothèse connue est réalisée au moyen d'un anneau de fixation de l'oreillette gauche qui entoure cette dernière et qui est fixé sur le boîtier du ventricule gauche bloquant la plaque qui sert de support aux valvules auriculo-ventriculaires gauches, une rondelle assurant la portée nécessaire à la liaison entre le boîtier du ventricule et l'anneau de fixation de l'oreillette.

La présente invention s'est en conséquence donné pour but de pourvoir à une nouvelle prothèse cardiaque totale qui répond mieux aux nécessités de la pratique que les prothèses cardiaques proposées dans l'Art Antérieur, notamment ce qu'elle présente un encombrement plus faible que les prothèses proposées dans l'Art Antérieur, tout en autorisant de grands débits, assurant ainsi de hautes performances ; en ce qu'elle utilise, de façon optimale, du fait de son encombrement relativement faible, le volume physiologiquement utilisable de la cavité péricardique et élimine pratiquement tout espace mort anatomique ; en ce qu'elle peut, le cas échéant, n'être activée que par une seule source d'activation pour les ventricules droit et gauche ; en ce qu'elle présente une géométrie fonctionnelle qui se rapproche au maximum de la géométrie fonctionnelle du cœur naturel, favorisant ainsi l'écoulement du sang dans la prothèse et évitant sa coagulation ; en ce qu'elle favorise la circulation de retour ; en ce que sa conception repose non pas seulement sur les variations du débit par l'intermédiaire des variations du volume systolique, mais aussi sur des variations de débit déterminées par l'augmentation de la fréquence d'activation de la prothèse ; et en ce que sa conception tient compte non seulement de la Loi de STARLING selon laquelle le volume systolique est fonction de la pression de remplissage, mais aussi de la Loi de SARNOFF selon laquelle les variations de débit s'accompagnent de variations proportionnelles de la force utilisée, l'augmentation du débit sanguin dans la prothèse ainsi déterminée contribuant à une éjection plus complète de sang du ventricule dans l'aorte (augmentation de puissance en fonction des besoins).

La présente invention a pour objet une prothèse cardiaque totale comprenant :

a) un boîtier rigide réalisé en matériau étanche compatible et non toxique vis-à-vis des tissus environnants, ledit boîtier délimitant un module cardiaque monobloc biventriculaire présentant une forme et un volume péricardique, exactement en les lieu et place du cœur naturel,

b) un dispositif moteur logé dans le boîtier et comportant deux membranes souples dont l'une, en élastomère, délimite avec la face interne correspondante du boîtier située en regard, un espace qui constitue le ventricule droit, tandis que l'autre, non élastique et de surface sensiblement inférieure à celle de la première membrane, délimite avec la face interne correspondante du boîtier située en regard, un espace ventriculaire gauche, la membrane élastique épousant la forme et s'appliquant sur la surface convexe d'un dispositif de soutien rigide dans sa condition non opératoire, tandis que la membrane non élastique est fixée périphériquement sur un support rigide,

c) des valves montées dans des orifices valvulaires du boîtier par l'intermédiaire d'inserts destinés à l'anastomose avec les vaisseaux du réseau circulatoire, lesdits inserts étant conçus de manière à conférer à chaque valve un diamètre intérieur sensiblement identique à celui de l'orifice valvulaire dans lequel elle est montée,

d) des moyens d'isolement du sang par rapport au boîtier et au dispositif moteur, lesdits moyens étant constitués par des poches ou sacs à sang en matériaux hémocompatibles isolants, un premier sac à sang étant logé dans l'espace ventriculaire droit de manière à être comprimé par l'élongation de la membrane élastique et un second sac à sang étant logé dans l'espace ventriculaire gauche de manière à être comprimé par le déplacement de la membrane non élastique de part et d'autre du plan de son support rigide périphérique, lesdits moyens d'isolement étant maintenus en place dans leurs espaces ventriculaires respectifs par fixation aux valves,

e) des moyens d'activation du dispositif moteur comportant au moins une source d'énergie qui génère une pression de fluide dans une chambre d'activation délimitée par les deux membranes logeant entre elles le dispositif de soutien de la membrane élastique, ladite pression d'activation provoquant une élongation de ladite membrane élastique entraînant des déplacements de volume dans l'espace ventriculaire droit, le volume déplacé par la membrane étant apte à être modifié en faisant varier la pression d'activation susdite, tandis que les déplacements de volume dans l'espace ventriculaire gauche sont obtenus par flexion de la membrane non-élastique sous l'effet de ladite pression, la différence d'action de la pression d'activation sur la membrane élastique, qu'elle soumet à une plus ou moins grande élongation en fonction de sa valeur, provoquant une activation asymétrique des ventricules droit et gauche et déterminant une variation correspondante du volume d'éjection du ventricule droit, et,

f) des moyens de régulation du débit sanguin du module en fonction des variations des résistances pulmonaire et systématique du porteur, ces moyens de régulation se composant de moyens de réglage de la pression fluidique d'activation et du pourcentage de systole dans le cycle d'activation en fonction de la pression aortique ainsi que de moyens de réglage de la durée du cycle d'activation en fonction de la pression de remplissage du ventricule gauche, l'ensemble de ces moyens de réglage agissant en synergie.

Selon un mode de réalisation de la prothèse cardiaque totale conforme à l'invention, la membrane en matériau élastomère qui travaille à l'élongation présente une forme convexe et une grande surface, de l'ordre de 150 à 165 cm$^2$.

Conformément à une disposition de l'invention, le dispositif de soutien rigide de la membrane élastique qui travaille à l'élongation joue à l'égard de ladite membrane le rôle de butée anti-retour.

Conformément à une autre disposition de l'invention, ledit dispositif de soutien rigide de la membrane élastique qui travaille à l'élongation, est percé d'au moins une ouverture qui assure la communication de fluide à travers celui-ci.

Selon un mode de réalisation de la prothèse conforme à la présente invention, l'ensemble constitué par les deux membranes susdites et leurs dispositifs de soutien, forme un ensemble étanche lui-même logé dans ledit boîtier en matériau étanche, auquel ledit ensemble est solidarisé par l'intermédiaire de logements appropriés ménagés dans ledit boîtier pour recevoir les extrémités des deux membranes et les extrémités de leurs supports respectifs, formant ainsi le « module cardiaque ».

Selon un mode de réalisation de la prothèse cardiaque totale conforme à la présente invention, la distance entre les membranes qui délimitent le ventricule gauche et le ventricule droit et la face interne correspondante dudit boîtier, est croissante dans les trois dimensions spatiales, de la pointe vers la base de la prothèse, pour orienter les vecteurs vitesse du sang vers l'orifice valvulaire de sortie correspondant et éviter toutes stases.

Selon encore un autre mode de réalisation de la prothèse cardiaque totale conforme à la présente invention, le volume de l'espace ventriculaire gauche et le volume de l'espace ventriculaire droit sont sensiblement égaux, tout en présentant une géométrie différente.

Selon un autre mode de réalisation de la prothèse cardiaque totale conforme à la présente invention, chacune des valves montées dans les orifices valvulaires ménagés dans le boîtier, est constituée par un disque en matériau hémocompatible approprié, dont le diamètre est sensiblement identique à celui de l'orifice valvulaire dans lequel il est monté, lequel disque est inséré entre une barrette d'articulation et deux barrettes de butée qui le bloquent en position ouverte ou fermée, en coopération avec ladite barrette d'articulation, créant, en position ouverte, une surface utile maximale et offrant une inertie minimale.

Selon encore un autre mode de réalisation de la prothèse cardiaque totale conforme à la présente invention, les inserts sont conçus pour recevoir respectivement l'extrémité de fixation de la barrette d'articulation et les extrémités de fixation des barrettes de butée, entre lesquelles est insérée la valve susdite et pour recevoir les extrémités des poches à sang qui sont fixées par pincement entre lesdits inserts et les moyens d'anastomose au porteur de la prothèse.

Selon un autre mode de réalisation de la prothèse conforme à l'invention, les moyens d'activation du dispositif moteur sont constitués par un moteur électrique à piles implantable, dont l'énergie fournit les pressions d'alimentation requises au dispositif moteur susdit.

Selon une autre disposition de l'invention, les moyens d'activation du dispositif moteur sont constitués par un moteur électrique commandé par une source d'énergie nucléaire, qui fournissent les pressions d'alimentation requises au dispositif moteur susdit et sont tous deux implantables.

Selon encore une autre disposition de l'invention, les moyens d'activation du dispositif moteur sont constitués par une source d'énergie pneumatique extra-corporelle reliée à la prothèse cardiaque par un tube transcutané de petit diamètre, réalisé en un matériau éliminant au maximum les risques d'infection transcutanée.

Compte tenu de sa structure monobloc, le module cardiaque conforme à la présente invention est avantageusement activé par une seule source d'activation pour les ventricules droit et gauche ; il peut cependant s'avérer commode, dans certains cas, notamment lorsque la source d'activation est pneumatique, de disposer, conformément à l'invention, de deux pompes indépendantes agissant respectivement l'une sur la membrane élastique du ventricule droit, l'autre sur la membrane déformable du ventricule gauche.

Selon un mode de réalisation de la prothèse conforme à la présente invention, les moyens de régulation du débit sanguin fonctionnant par asservissement de la pression fluidique d'activation et du pourcentage de temps de systole dans le cycle d'activation, à une valeur de référence de la pression aortique moyenne, ainsi que par réglage de la durée du cycle d'activation en fonction de la pression de remplissage dans le ventricule gauche, limitent la fréquence d'activation à des valeurs comprises entre 80 et 110 cycles/minute.

Selon une disposition de l'invention, lesdits moyens de régulation du débit cardiaque comprennent, dans le cas où ils coopèrent avec des moyens d'activation constitués par une source d'énergie pneumatique : un premier asservissement qui comporte un dispositif mécanique agissant sur un détendeur de pression qui fait varier la pression d'activation entre les valeurs limites de 306 et 440 millibars, sous l'action d'un signal émis par un comparateur, après comparaison d'un signal correspondant à la pression aortique effective du porteur de la prothèse, avec un signal correspondant à une pression de référence, de 133, 326 millibars. Si la pression effective diffère de la pression de référence, la pression exercée sur la membrane qui délimite le ventricule droit augmente ou diminue et le débit dans ledit ventricule droit varie de façon correspondante ; — et un deuxième asservissement qui utilise l'augmentation du débit dans le ventricule droit et l'augmentation résultante de la pression de remplissage du ventricule gauche, qui est représentée par un signal de pression représentatif de la pression de remplissage du ventricule gauche, pour réguler la période d'activation entre 750 et 530 millisecondes à l'aide d'un signal proportionnel émis à la suite de l'établissement par un microprocesseur, d'une relation proportionnelle entre la pression de remplissage du ventricule gauche et la période du cycle d'activation.

La régulation en fonction de la pression aortique est donc réalisée conformément aux dispositions qui précèdent, en comparant un signal correspondant à la pression aortique à un signal de référence correspondant à une pression aortique prédéterminée, par exemple 133, 326 millibars, le signal de sortie du comparateur commandant la pression du fluide pour faire varier de façon correspondante la pression d'activation sur la membrane d'activation du ventricule droit et, par voie de conséquence, pour faire varier le débit dans ce ventricule, le choix du signal de référence, c'est-à-dire de la pression aortique de référence, correspondant à un volume circulant et à un débit de sang normaux pour le patient dans les conditions de repos. Or, le volume circulant peut varier en fonction des conditions physiologiques telles que l'alimentation du patient. Dans le cas de l'absorption de boissons, en particulier, le volume circulant augmente et la pression aortique du cœur normal augmente afin d'éliminer cet excès de liquide par perfusion à travers les reins.

Cette régulation du volume circulant est une caractéristique peu connue du cœur, dont il est apparu nécessaire de tenir compte pour affiner la régulation de la prothèse cardiaque conforme à la présente invention.

En conséquence, selon une autre caractéristique avantageuse du dispositif de régulation du débit sanguin de la prothèse conforme à la présente invention, celui-ci comprend un troisième asservissement destiné à faire varier la pression aortique en fonction du volume circulant, par détection de la pression de remplissage du ventricule droit et comparaison de cette pression avec une pression de référence, le résultat de cette comparaison commandant la valeur de la pression de référence déterminée de l'asservissement à la pression aortique.

La variation de volume ainsi obtenue correspond à une faible variation de même sens de la pression de remplissage du ventricule droit.

Pour éviter les variations accidentelles, les mesures des pressions de remplissage du ventricule droit sont enregistrées pendant une durée prédéterminée et c'est la moyenne des mesures enregistrées qui est comparée à la pression de référence.

La présente invention a également pour objet le dispositif de régulation du débit sanguin dans la prothèse cardiaque, qui comprend un appareillage électronique associé à des moyens mécaniques, et qui est du type comprenant deux moyens d'asservissement, lequel dispositif est caractérisé en ce que le premier moyen d'asservissement établit une relation directe entre la pression aortique moyenne d'une part, et d'autre part la pression systolique d'activation et le pourcentage du temps de systole dans le cycle, tandis que le deuxième moyen d'asservissement établit une relation entre la pression moyenne de remplissage du ventricule gauche et la fréquence cardiaque, une relation indirecte s'établissant ainsi entre le premier et le deuxième moyens d'asservissement qui agissent en synergie, en raison du fait que le premier moyen d'asservissement modifie la pression moyenne de remplissage du ventricule gauche en faisant varier le volume systolique droit et le deuxième moyen d'asservissement fait varier la fréquence cardiaque lorsque varie la pression moyenne de remplissage du ventricule gauche.

Selon un mode de réalisation avantageux du dispositif de régulation du débit sanguin dans une prothèse cardiaque, ledit dispositif comprend, en outre, un troisième asservissement qui commande des variations de la pression aortique en fonction des variations du volume du sang circulant, lesquelles se traduisent par une variation de la pression de remplissage du ventricule droit, dont la mesure et la comparaison avec une pression moyenne prédéterminée commandent la variation de la pression aortique initiale.

Outre les dispositions qui précèdent, l'invention comprend encore d'autres dispositions qui ressortiront de la description qui va suivre.

La présente invention vise plus particulièrement les prothèses cardiaques conformes aux dispositions qui précèdent, ainsi que les moyens mis en œuvre pour leur fabrication et leur réalisation.

L'invention sera mieux comprise à l'aide du complément de description qui va suivre, qui se réfère aux dessins annexés dans lesquels :

— la figure 1 est une vue en coupe longitudinale d'un module cardiaque conforme à la présente invention ;

— la figure 2 est une vue en coupe transversale du module de la figure 1 ;

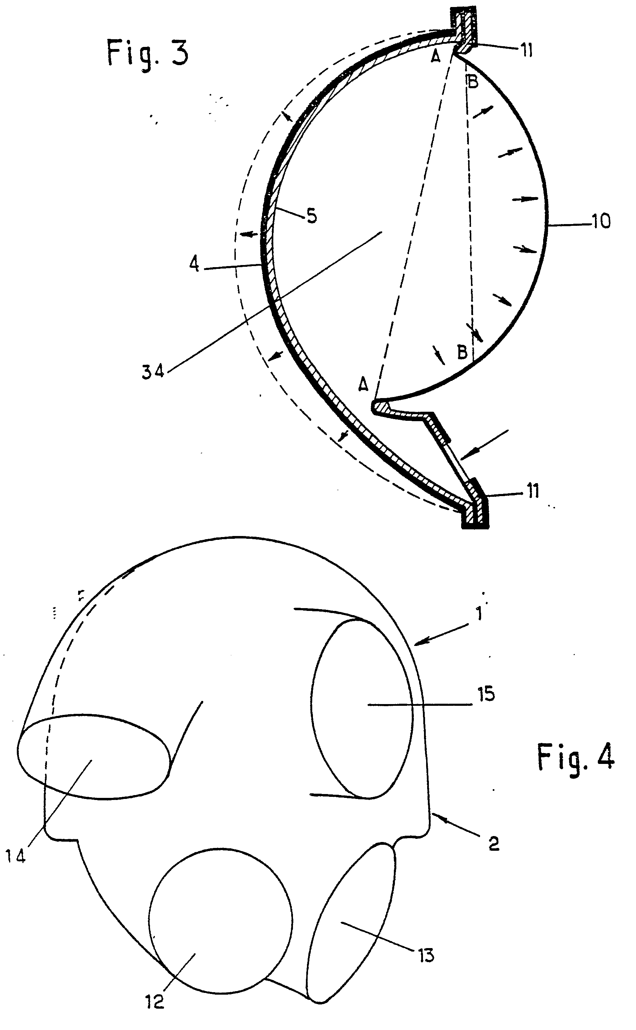

— la figure 3 est une vue en coupe longitudinale du dispositif moteur de la prothèse conforme à l'invention ;

— la figure 4 est une vue schématique en perspective du boîtier de la prothèse conforme à l'invention comportant quatre orifices valvulaires ;

— la figure 5 est une vue de face d'une valve conforme à l'invention représentée en position ouverte ;

— la figure 6 est une vue en coupe, à plus grande échelle, d'un insert destiné à être monté dans les orifices valvulaires du boîtier pour recevoir la valve représentée à la figure 5 ;

— les figures 7 et 8 représentent les schémas des asservissements qui réalisent la régulation du débit sanguin dans la prothèse, et

— la figure 9 est un schéma du troisième asservissement inclus dans le dispositif de régulation du débit sanguin conforme à l'invention.

Il doit être bien entendu, toutefois, que ces dessins et les parties descriptives correspondantes, sont donnés uniquement à titre d'illustration de l'objet de l'invention, dont ils ne constituent en aucune manière une limitation.

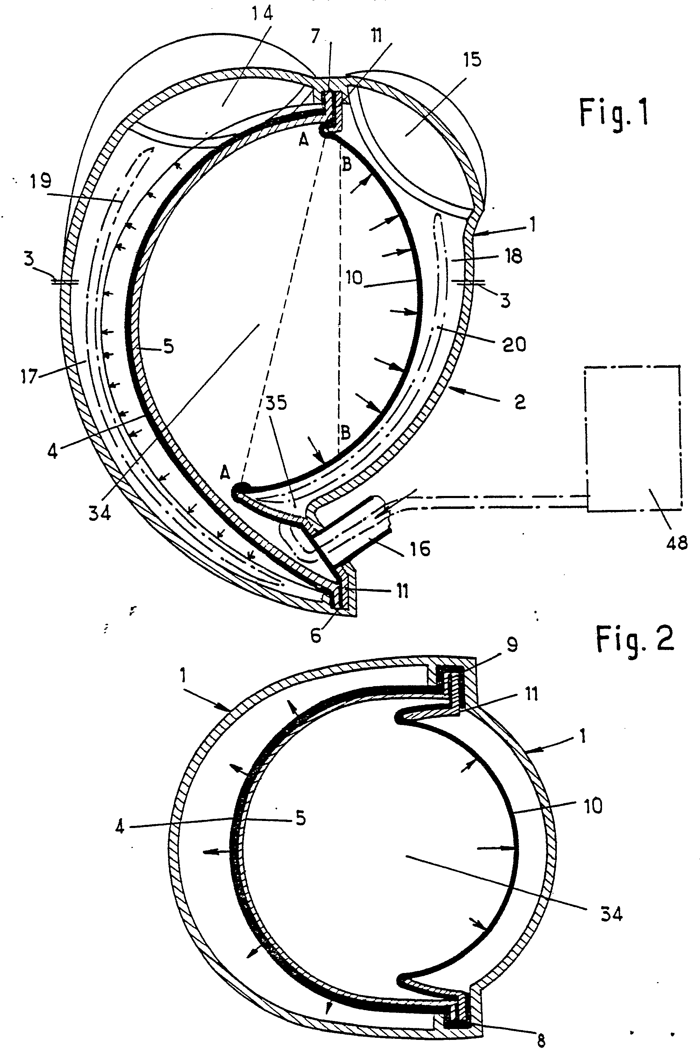

Le module de la prothèse cardiaque conforme à la présente invention est constitué par un ensemble monobloc biventriculaire qui comprend un boîtier réalisé en un matériau non toxique à l'égard des tissus environnants tel qu'un polyuréthane par exemple, lequel boîtier comprend une partie supérieure 1 et une partie inférieure 2 réunies l'une à l'autre de façon étanche, par des clips 3, par exemple, dans un plan perpendiculaire aux directions de travail du dispositif moteur qui sera décrit plus loin. Ce boîtier 1, 2 renferme une membrane 4 en matériau élastomère, qui travaille à l'élongation, et qui présente une grande surface, de l'ordre de 150 à 165 cm², et qui délimite le ventricule droit de la prothèse avec la face interne de la partie droite de la paroi du boîtier 1, 2 ; la forme de la membrane 4 est telle que l'espace ventriculaire droit s'élargit régulièrement dans les trois dimensions de l'espace, de la pointe vers la base, lorsque la membrane 4 est mise en position opérationnelle. Cette membrane 4 en élastomère présente des caractéristiques dynamiques très spécifiques : le déplacement de volume varie de 80 à 170 ml lorsqu'elle est soumise à une variation de pression de 306 à 440 millibars pendant une durée de 240 millisecondes. Cette membrane 4 est supportée par un dispositif de soutien 5 constitué par une plaque en matériau rigide tel qu'acier inoxydable par exemple, qui épouse exactement la forme de la membrane 4 qui s'applique sur la face externe du dispositif de soutien 5. Celui-ci est fixé au boîtier 1, 2, conjointement avec la membrane 4, dans un logement 6, 7, 8, 9 du boîtier, prévu à cet effet, lequel s'étend sur toute la hauteur de la prothèse, de la pointe à la base de cette dernière. La plaque 5 est avantageusement percée de trous sur la totalité de sa surface pour assurer la communication de l'air distribué par une source d'énergie pneumatique reliée à la prothèse, avec la membrane qui délimite le ventricule gauche. Une deuxième membrane, 10, est également montée dans le boîtier 1, 2 ; cette membrane 10, qui délimite le ventricule gauche avec la face interne de la partie gauche du boîtier 1, 2, travaille à la flexion (et non pas à l'élongation comme la membrane 4) de part et d'autre d'un plan BB qui fait un angle de 15° par rapport au plan axial longitudinal AA de la prothèse. La membrane 10 est fixée latéralement sur un support 11 en matériau rigide tel qu'acier inoxydable par exemple, et le support 11, avec la partie correspondante de la membrane 10, sont fixés dans les logements 6, 7, 8, 9 correspondants du boîtier 1, 2. Des orifices valvulaires 12, 13, 14, 15 sont ménagés dans le boîtier 1, 2, lequel est connecté, par une connexion désignée d'une façon générale par la référence 16 à la figure 1, à une source d'énergie pneumatique (non représentée), par l'intermédiaire d'un tube transcutané (également non représenté) de petit diamètre (10-12 mm par exemple) recouvert de tissu réhabitable et comportant à son extrémité un disque en pyrolyte de carbone placé sous la peau, qui empêche le cisaillement axial du tube sur la peau et évite les infections.

Le montage des membranes 4 et 10 dans le boîtier 1, 2, est tel que l'espace ventriculaire droit 17, qui sépare la membrane 4 de la face interne de la paroi droite du boîtier 1, 2, est croissant dans les trois dimensions de l'espace, de la pointe vers la base de la prothèse. De même, l'espace 18 qui sépare la membrane 10 de la face interne de la paroi gauche du boîtier 1, 2, est également croissant dans les trois dimensions de l'espace, de la pointe vers la base de la prothèse. Une telle disposition a pour effet de favoriser des écoulements laminaires, sans turbulences, et d'éliminer les zones de vitesses nulles dans les espaces ventriculaires 17 et 18, tout en orientant dans les espaces ventriculaires 17 et 18, les vecteurs vitesse du sang vers la sortie, c'est-à-dire vers l'aorte et l'artère pulmonaire, respectivement. Une mince poche 19, 20, en matériau hémocompatible, tel que polyuréthane polysegmenté, par exemple, isole le dispositif moteur constitué par les membranes 4 et 10 et le boîtier, du sang circulant dans les ventricules 17, 18 de la prothèse conforme à l'invention. Les volumes des ventricules droit 17 et gauche 18 sont sensiblement égaux et sont respectivement de l'ordre de 250 cm³.

Des inserts, désignés d'une façon générale par la référence 21, sont montés dans le boîtier 1, 2, et plus spécifiquement dans les orifices valvulaires 12, 13, 14, 15, qui sont respectivement les orifices aortique, mitral, pulmonaire et tricuspide, un peu au-dessous du plan de l'embouchure desdits orifices.

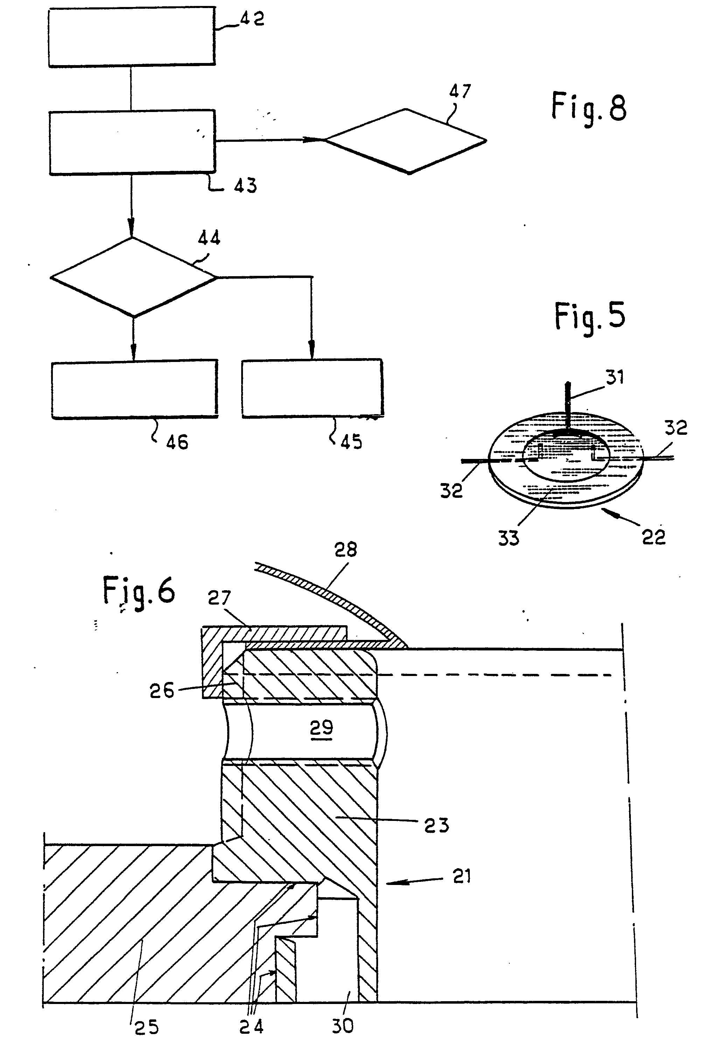

Ces inserts 21, dont l'un a été représenté à l'échelle très agrandie à la figure 6, approximativement à l'échelle 10, sont des pièces qui réalisent la jonction entre le module cardiaque et le porteur de la prothèse, et qui permettent la mise en place des valves 22 de la prothèse conforme à l'invention dans les orifices valvulaires, d'une manière qui réduit considérablement les pertes de charge. L'insert 21 représenté à la figure 6 comporte un corps 23, en acier inoxydable par exemple. Un logement 24 de l'insert reçoit à force une section correspondante 25 du bord de l'orifice valvulaire qui reçoit l'insert, cette section s'adaptant dans ledit logement 24. Le corps 23 de l'insert comporte en outre un filetage latéral 26 qui reçoit une pièce de jonction 27 qui porte un disque en « Teflon » (marque déposée) 28 pour l'anastomose au porteur de la prothèse. Le corps 23 comporte en outre un alésage 29 et deux alésages 30. L'alésage 29 est destiné à recevoir l'une des extrémités de la barrette d'articulation 31 d'une valve désignée d'une façon générale par la référence 22, tandis que les alésages 30 sont destinés à recevoir une des extrémités de chacune des barrettes de butée 32 de ladite valve ; le disque 33 de la valve, qui constitue la valve proprement dite, est inséré entre les extrémités libres des barrettes 31 et 32. Il est réalisé de préférence en matière plastique thermoplastique hémocompatible appropriée ; son diamètre est sensiblement identique à celui des orifices valvulaires auxquels il est associé et est également sensiblement identique à celui des vaisseaux auxquels la prothèse doit être anastomosée. En raison du montage des extrémités des barrettes de la valve 22 dans les inserts 21, et de l'élimination de l'anneau de support métallique dont sont pourvues les valves connues dans l'Art antérieur, les causes de restriction du débit (pertes de charge) sont éliminées dans une grande mesure, de même que, dans une mesure importante, les causes de thrombogénèse.

En service, les déplacements de volume dans le ventricule droit sont obtenus en sollicitant, sous l'action de l'air (ou de l'hélium) comprimé provenant du distributeur pneumatique, plus ou moins l'élongation de la membrane 4 élastique convexe, qui figure le septum interventriculaire du cœur naturel ; en raison de la grande surface de la membrane 4, l'amplitude utile est faible et les contraintes mécaniques imposées à l'élastomère qui constitue la membrane 4 sont négligeables, attendu que l'élastomère qui constitue la membrane 4, qui travaille en étirement périodique, ne subit qu'une élongation de l'ordre de 10 à 15 % en raison de la surface importante de la membrane, en sorte que la fatigue à laquelle il est soumis est pratiquement nulle. Les déplacements de volume dans le ventricule gauche sont obtenus par flexion de la membrane non élastique 10, de part et d'autre du plan BB de la prothèse.

Une prothèse cardiaque doit pomper tout le sang qui lui parvient du réseau circulatoire sans que les pressions moyennes d'alimentation des ventricules s'écartent de leurs valeurs physiologiques qui sont de — 2,66 à + 9,33 millibars pour le ventricule droit et de + 6,66 à + 20 millibars pour le ventricule gauche.

Comme le débit de sang qui parvient au cœur est inversement proportionnel à la valeur des résistances du territoire systémique, le débit pompé par le cœur dans ce territoire doit être également inversement proportionnel à ces résistances pour que la pression artérielle soit maintenue à sa valeur physiologique.

Pour ce faire, chez un sujet jeune de 80 kg, la sensibilité du ventricule gauche à la pression de remplissage doit être de l'ordre de 1 litre par 1,333 millibar et par minute, ce qui correspond à des variations de débit de l'ordre de 6 à 19 litres par minute.

Pour reproduire avec une prothèse cardiaque ces performances qui sont celles du cœur naturel lorsque l'organisme passe des conditions de repos (consommation d'oxygène 400 cm³/minute) à celles d'un exercice musculaire d'intensité maxima (consommation d'oxygène 1 300 cm³/minute), trois types d'adaptation sont possibles. Une adaptation de débit basée sur les seules variations du volume systolique sous l'effet des variations de la pression de remplissage ; une adaptation de débit basée sur des variations de fréquence ; une adaptation de débit comportant comme le cœur naturel, ces deux mécanismes mis en jeu de façon synergique.

Une autorégulation de débit basée uniquement sur les variations du volume systolique telle qu'elle est réalisée actuellement par la plupart des prothèses connues est insuffisante pour la raison suivante : la fréquence utilisable en pratique de façon prolongée est de 90 à 110 cycles par minute ; l'autorégulation de débit implique donc un grand volume de réserve diastolique qui n'est pas compatible avec les contraintes anatomiques. De plus, les valves d'admission jusqu'ici utilisées ne dépassent pas 30 mm de diamètre interne pour des raisons d'encombrement également. Le temps nécessaire à l'éjection du volume de sang contenu dans la ventricule étant de 35 à 40 % de la période d'activation du cœur, le temps laissé libre pour le remplissage dans le cycle n'est que de l'ordre de 340 à 400 millisecondes. Or le volume maximum de sang qui peut passer pendant ce temps de l'oreillette dans le ventricule lorsque la pression moyenne de remplissage est à son plus haut niveau acceptable (20 millibars) n'est dans ces conditions que de 110 à 130 ml (filling impédance).

Les facteurs limitant le débit lorsqu'on fonctionne à fréquence fixe sont donc d'une part les contraintes anatomiques qui limitent le volume de réserve disponible, et, d'autre part, les pertes de charges au niveau de la valve d'admission qui limitent l'utilisation de ce volume.

En pratique, pour un encombrement global effectif supérieur à 800 cm³, les prothèses connues dans l'Art antérieur effectuent sous une pression de remplissage de 20 millibars, un débit inférieur à 13 litres/minute.

D'autres investigateurs ont tenté de réaliser une régulation de débit par l'intermédiaire de variations de fréquence appropriées. Ces prothèses fonctionnent à volume systolique constant, un dispositif électronique arrêtant la phase de remplissage dès que les ventricules sont pleins, sous l'effet d'un signal de contact. Ces prothèses étant faites de deux parties indépendantes, droite et gauche, les phénomènes d'encombrement limitent le volume systolique maximum à 130 ml.

Il est apparu que l'association, conformément à l'invention, d'une prothèse monobloc présentant un faible encombrement (700 cm³), de grands volumes de réserve (190 ml), de grandes valves d'admission (35 mm de diamètre interne) avec un dispositif de régulation portant à la fois sur les variations du volume systolique (STARLING), sur la fréquence et sur la puissance d'éjection (SARNOFF) permet de couvrir des variations de débit plus étendues en respectant les pressions de remplissage physiologiques indiquées ci-dessus et, par voie de conséquence, assure une meilleure régulation de la pression artérielle. Conformément à l'invention, un tel dispositif commande la période de la phase de remplissage en fonction de la pression de remplissage du ventricule gauche et la pression d'activation en fonction des résistances du territoire systémique du sujet.

Conformément à l'invention, ce dispositif de régulation comprend deux asservissements, à savoir :

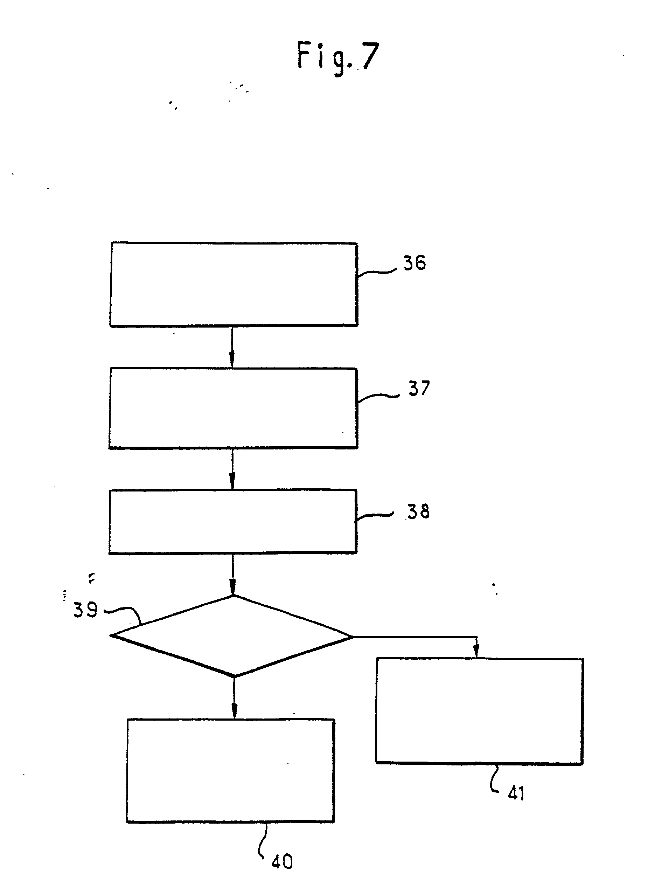

Un premier asservissement (cf. figure 7) établit une relation entre, d'une part, la pression d'activation utilisée sur le distributeur de pression pneumatique et le pourcentage du temps de systole, et, d'autre part, une valeur de référence de la pression aortique moyenne. La pression aortique moyenne prise comme référence est celle du patient avant l'implantation de la prothèse, soit 120 à 160 millibars. La pression aortique moyenne effective est repérée grâce à l'analyse morphologique de la courbe de montée en pression de l'air capté dans le tube d'activation, le plus près possible de la prothèse. Cette courbe permet de déterminer : (1) l'ouverture de la valve de sortie du ventricule gauche et la pression correspondante, qui est la pression diastolique du sujet ; (2) à partir du décrochage correspondant à l'ouverture de ladite valve, la courbe de pression s'infléchit et correspond à l'éjection de tout le contenu du ventricule gauche dans l'aorte ; le sommet de cette phase correspond à la pression « systolique » du sujet ; (3) la courbe se redresse ensuite pour atteindre la valeur de pression utilisée sur le distributeur de pression pneumatique. Lorsque le temps de systole est supérieur à 240 millisecondes, lorsque la pression aortique moyenne du sujet ne dépasse pas 160 millibars et lorsque la pression d'activation est supérieure à 306 millibars, cette phase de la courbe est toujours apparente.

Comme la membrane d'activation 10 du ventricule gauche n'est pas élastique, la pente de la courbe à partir du deuxième décrochage ne dépend que de la résistance de la membrane 4 du ventricule droit à l'élongation, puisque la pression d'activation est commune aux ventricules droit et gauche. L'analyse morphologique de la courbe de pression d'activation permet de déterminer la pression diastolique et la pression systolique du sujet. De ce signal, on tire la pression aortique moyenne : ce signal pilote : — la pression d'activation entre des valeurs limites de 306 à 440 millibars ; — le pourcentage du temps de systole dans le cycle d'activation entre les valeurs limites de 33 % à 43 %. Si la pression aortique moyenne mesurée est supérieure ou inférieure à la valeur choisie comme référence, la pression d'activation doit augmenter ou diminuer en sens inverse d'une valeur de 13,33 millibars ; le pourcentage du temps de systole dans le cycle doit augmenter ou diminuer en sens inverse, également de 1 %. Une variation de la pression aortique moyenne au-dessus ou au-dessous de la valeur de référence entraîne donc une variation en sens inverse de la pression d'activation et du pourcentage du temps de systole dans le cycle dans les limites indiquées ci-dessus. Ceci a pour effet d'augmenter ou de diminuer le volume déplacé par le ventricule droit et donc le débit d'alimentation du ventricule gauche.

Ce premier asservissement effectue donc trois opérations : (1) extraction du signal pilote sur la courbe de montée en pression de l'air, de préférence à 25 cm de la prothèse ; (2) traitement de ce signal pour moduler le pourcentage du temps de systole dans le cycle dans les valeurs limites de 33 à 43 % ; le prélèvement du signal pilote est effectué toutes les vingt secondes ; (3) mise en action d'un dispositif mécanique agissant sur un détendeur de pression associé au distributeur de pression pneumatique, pour régler la pression dans la capacitance 48 dudit distributeur entre 306 et 440 millibars en fonction de la pression aortique moyenne mesurée chez le sujet. Cette opération a pour but de maintenir le temps de la systole à une valeur constante (240 milliesecondes ± 10 %) nécessaire à l'éjection de tout le volume contenu dans les ventricules.

Conformément au schéma représenté à la figure 7, le premier asservissement comporte un capteur de pression 36 placé à 25 cm de la prothèse, sur le tuyau d'activation 16, un dispositif électronique 37 de traitement du signal émis par le capteur 36, par l'étude morphologique de la courbe de montée en pression de l'air, un comparateur 38 qui capte le signal correspondant à la pression aortique moyenne du porteur de la prothèse et le compare au signal correspondant à la pression aortique de référence (133,326 millibars ± 13,33 millibars) et émet un signal de réglage de la pression d'activation : si la pression aortique effective moyenne comparée par le dispositif 38 est supérieure à la pression aortique de référence, le signal émis en 40 provoque, dans la capacitance 48, une diminution de la pression d'activation de 13,33 millibars (temps de systole : — 1 %), si la pression aortique effective moyenne comparée par le dispositif 38 est inférieure à la pression aortique de référence, le signal émis en 41 provoque, dans la capacitance 48, une augmentation de la pression d'activation de 13,33 millibars (temps de systole : + 1 %).

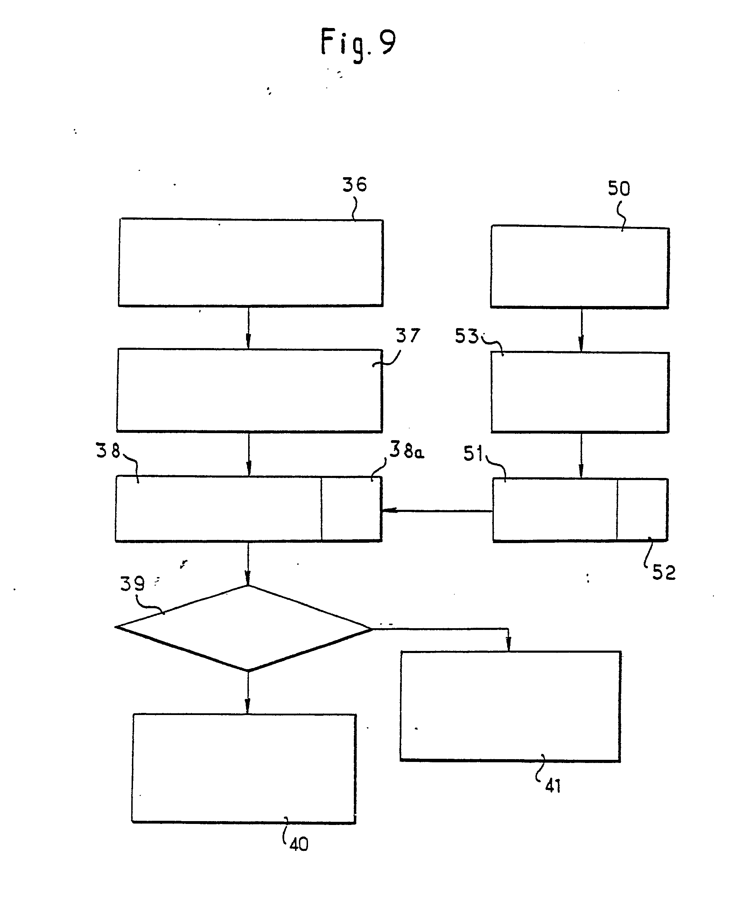

Un deuxième asservissement (cf. figure 8) établit une relation entre la période du cycle d'activation et la pression de remplissage du ventricule gauche.

La pression de remplissage du ventricule gauche est tirée du signal de pression capté dans une capacitance 48 extracorporelle, en communication avec l'espace intermembranaire 35 de la prothèse, par un tube 49 co-axial au tube 16 d'activation (diamètre interne 3 mm ; diamètre externe 4 mm ; longueur 30 cm). Pour réduire les phénomènes de pertes de charge, cette capacitance 48 peut contenir de l'hélium. Le signal représentatif de la pression de remplissage du ventricule gauche pilote la période du cycle d'activation dans les valeurs limites comprises entre 750 à 550 millisecondes.

A une pression moyenne de remplissage du ventricule gauche de 9,33 millibars correspond une fréquence de 95 cycles par minute (c'est-à-dire un cycle d'une durée de 630 millisecondes). Une augmentation de la pression moyenne de remplissage du ventricule gauche au-dessus de 9,33 millibars détermine une diminution de la durée du cycle de 30 millisecondes, jusqu'à une valeur limite de 550 millisecondes et une diminution de la pression moyenne de remplissage du ventricule gauche au-dessous de 9,33 millibars détermine une augmentation de la durée du cycle de 30 millisecondes jusqu'à une valeur limite de 750 millisecondes. Le prélèvement du signal dans la capacitance est effectué avec un délai de 10 secondes sur le prélèvement du signal du premier asservissement.

Ce deuxième asservissement a pour rôle de convertir le signal de pression obtenu en signal de temps.

Conformément au schéma représenté à la figure 8, le capteur de pression 42 placé dans la capacitance 48 capte un signal représentatif de la pression de remplissage du ventricule gauche, qui doit être égale à 9,33 millibars, la durée du cycle étant alors de 650 millisecondes. Un comparateur 44 compare le signal représentatif de la pression effective de remplissage du ventricule gauche à un signal représentatif de la pression de référence susdite : si le signal émis par le comparateur 44 constate une égalité (47) aucun signal de réglage de la durée du cycle n'est émis ; si la pression de remplissage est supérieure à la pression de remplissage de référence, un signal est émis en 46 pour augmenter la durée du cycle de 30 millisecondes ; dans le cas contraire, un signal émis en 45 provoque la diminution de la durée du cycle de 30 millisecondes, les valeurs limites de la durée du cycle étant de 550 millisecondes-750 millisecondes.

Le troisième asservissement, tel que représenté à la figure 9 est couplé au premier asservissement qui, comme on l'a décrit plus haut, établit une relation entre d'une part, la pression d'activation utilisée sur le distributeur de pression pneumatique et le pourcentage du temps de systole et, d'autre part, une valeur de référence de la pression aortique moyenne prédéterminée.

Cette pression aortique prédéterminée est celle du patient avant implantation de la prothèse, le patient étant dans un état normal, c'est-à-dire ayant un volume circulant constant. Or, ce volume ne peut être considéré comme constant. En effet, lors de l'alimentation solide et surtout liquide, une partie du liquide ingéré passe dans le sang, augmentant ainsi le volume total de celui-ci. Or, le mécanisme physiologique fait que cette augmentation de volume se traduit par une augmentation de la pression artérielle qui, en agissant sur le rein, provoque l'hémodialyse rénale qui élimine le surplus de liquide dans le sang et rétablit le volume initial du sang.

Pour tenir compte de ce fonctionnement régulateur du cœur, le dispositif régulateur de la prothèse doit agir pour augmenter la pression aortique quand le volume du sang augmente. La pression aortique prédéterminée du dispositif d'asservissement doit donc être modifiée.

L'asservissement en fonction de la pression aortique utilise le capteur de la pression aortique 36 et le dispositif électronique 37 de traitement du premier asservissement, dont le signal est envoyé au comparateur 38 pour être comparé au signal de pression aortique de référence engendré par un générateur 38a du signal de référence inclus dans ce comparateur 38. Le troisième asservissement doit donc agir sur ce générateur 38a pour commander la valeur de la pression aortique prédéterminée en fonction des variations du volume sanguin.

Ces variations du volume sanguin qui se traduisent par une variation de la pression de remplissage du ventricule droit, conduisent à mesurer cette pression de remplissage pour la comparer à une pression moyenne prédéterminée qui se situe aux alentours de 2,66 millibars, le résultat de cette comparaison commandant la variation de la pression aortique de référence initialement fixée aux alentours de 133,326 millibars.

Ainsi, le troisième asservissement conforme à la présente invention comporte un capteur 50 de la pression de remplissage du ventricule droit, par exemple un capteur piézoélectrique placé dans l'espace ventriculaire droit de la prothèse ou un capteur de pression placé dans une capacitance reliée par un tube à l'espace ventriculaire droit de la prothèse ; ce capteur envoie son signal de sortie vers un comparateur 51 pour le comparer à un signal de sortie d'un générateur 52 d'un signal de référence de la pression de remplissage du ventricule droit, le signal de sortie du comparateur commandant le générateur du signal de référence de la pression aortique.

Pour éviter qu'une variation intempestive et accidentelle fugitive de la pression de remplissage du ventricule droit n'influence la pression aortique, le signal de sortie du capteur 50 est envoyé à un intégrateur ou enregistreur 53 qui ne délivre un signal qu'au bout d'une période prédéterminée, une heure par exemple, ce signal intégré étant envoyé au comparateur 51 pour être comparé à un signal de référence correspondant.

Les asservissements conformes à l'invention agissent en synergie et sont réalisés à l'aide d'un microprocesseur, pour permettre à la prothèse cardiaque conforme à l'invention de répondre par des variations de débit appropriées, aux variations physiologiques des résistances du circuit vasculaire, dans le but de ramener constamment la pression aortique moyenne à sa valeur de référence, dans le cas où seuls les deux premiers asservissements sont prévus et de la faire varier en fonction de la variation du volume du sang circulant dans la prothèse, dans le cas où le dispositif de régulation du débit sanguin comporte également le troisième asservissement conforme à l'invention ; ces asservissements permettent en outre de maintenir la pression de remplissage ventriculaire gauche dans les limites physiologiques (9,33 millibars ± 9,33).

La combinaison de moyens qui caractérise l'invention et qui comprend : — un boîtier monobloc étanche dont la forme et l'encombrement spatial reproduisent sensiblement ceux du cœur naturel ; — un dispositif moteur étanche, inclus dans ledit boîtier et comprenant une membrane convexe de grande surface, travaillant à l'élongation, dont la sollicitation permet d'obtenir les déplacements de volume requis dans le ventricule droit délimité par cette membrane et la paroi correspondante du boîtier, selon des modalités spécifiques : une augmentation de la pression d'activation de 306 à 440 millibars entraîne une variation du volume déplacé de 80 à 170 ml ; et une membrane travaillant à la flexion de part et d'autre d'un plan de symétrie, dont les déformations provoquent les déplacements de volume requis dans le ventricule gauche délimité par cette seconde membrane et la partie correspondante du boîtier ; — l'activation de ce dispositif moteur par une source d'activation unique (ou éventuellement par deux pompes d'activation) ; — la disposition dans les orifices valvulaires ménagés dans le boîtier et dont le diamètre est suffisamment grand pour ne pas constituer une gêne pour la circulation, de valves dont la section de passage est suffisamment grande pour diminuer le plus possible les pertes de charges trans-valvulaires ; une telle combinaison de moyens permet de conférer à la prothèse cardiaque conforme à la présente invention des dimensions optimales en égard à son encombrement, à l'absence de gêne concernant la circulation de retour et à la réduction des espaces morts anatomiques et fonctionnels à un minimum, tout en lui permettant de fonctionner à des débits sanguins sensiblement supérieurs à ceux que permettent d'assurer les prothèses cardiaques connues dans l'Art antérieur. C'est ainsi que le volume global de la prothèse conforme à la présente invention ne dépasse pas 700 cm$^3$, tandis qu'elle est capable d'assurer des débits maximums pouvant atteindre 18,5 à 19 litres/minute à une fréquence de 100 cycles/minute, à partir d'une pression moyenne du ventricule gauche de 20 millibars. L'encombrement des prothèses connues dans l'Art antérieur est compris entre 700 et 850 cm$^3$ et les débits maximums qu'elles sont capables de pomper sont de l'ordre de 12 à 14 litres, 14 litres étant au maximum, à une fréquence de 110 cycles/minute et à une pression moyenne de 20 millibars. L'encombrement et les performances de la prothèse cardiaque conforme à l'invention répondent donc parfaitement, comme on l'a montré plus haut, aux contraintes anatomiques et physiologiques dont le respect est une condition sine qua non du succès de l'implantation d'une prothèse cardiaque. En raison de sa structure monobloc biventriculaire, la prothèse cardiaque conforme à l'invention s'implante comme une greffe cardiaque.

Viennent s'ajouter aux caractéristiques avantageuses précitées, les dispositions prises pour minimiser les risques de thrombogénèse, par la suppression des pièces métalliques en contact avec le sang et par le choix des matériaux utilisés pour la réalisation des poches qui isolent la circulation sanguine dans les espaces ventriculaires 17 et 18 à l'égard du dispositif moteur et du boîtier. Vient enfin s'ajouter aux caractéristiques précitées, la disposition de moyens de régulation qui, conformément à l'invention, permettent la régulation des débits sanguins dans la prothèse cardiaque non seulement en fonction des pressions de remplissage, qui sont des pressions d'entrée, d'amont, mais aussi en fonction des pressions aortiques et pulmonaires, qui sont des pressions de sortie, d'aval, permettant ainsi l'adaptation des débits sanguins dans la prothèse aux variations des résistances pulmonaires et systémiques ; la pression d'activation et la durée de la systole sont asservies à une valeur de référence de la pression aortique moyenne et la fréquence du cycle d'activation est asservie à la pression du ventricule gauche, dans les valeurs limites de 80 à 100 cycles/minute. Ces deux asservissements agissant en synergie, permettent à la prothèse de répondre aux variations des résistances pulmonaires et systémiques par des variations de puissance (« contractilité »), qui préviennent l'augmentation de la pression veineuse centrale et reproduisent en cela le mode d'adaptation de débit du type homéométrique décrit par SARNOFF.

## Revendications

1. Prothèse cardiaque totale comprenant :

a) un boîtier rigide (1, 2) réalisé en matériau étanche compatible et non toxique vis-à-vis des tissus environnants, ledit boîtier délimitant un module cardiaque monobloc biventriculaire présentant une forme et un volume permettant son implantation dans l'espace péricardique, exactement en les lieu et place du cœur naturel,

b) un dispositif moteur logé dans le boîtier (1, 2) et comportant deux membranes souples (4, 10) dont l'une (4), en élastomère, délimite avec la face interne correspondante du boîtier (1, 2) située en regard, un espace qui constitue le ventricule droit (17), tandis que l'autre (10), non élastique et de surface sensiblement inférieure à celle de la première membrane (4), délimite avec la face interne correspondante du boîtier (1, 2) située en regard, un espace ventriculaire gauche (18), la membrane élastique (4) épousant la forme et s'appliquant sur la surface convexe d'un dispositif de soutien rigide (5) dans sa condition non opératoire, tandis que la membrane non élastique (10) est fixée périphériquement sur un support rigide (11),

c) des valves (22) montées dans des orifices valvulaires (12, 13, 14, 15) du boîtier par l'intermédiaire d'inserts (21) destinés à l'anastomose avec les vaisseaux du réseau circulatoire, lesdits inserts étant conçus de manière à conférer à chaque valve (22) un diamètre intérieur sensiblement identique à celui de l'orifice valvulaire dans lequel elle est montée,

d) des moyens d'isolement du sang par rapport au boîtier et au dispositif moteur, lesdits moyens étant constitués par des poches ou sacs à sang en matériaux hémo-compatibles isolants, un premier sac à sang (19) étant logé dans l'espace ventriculaire droit (17) de manière à être comprimé par l'élongation de la membrane élastique (4) et un second sac à sang (20) étant logé dans l'espace ventriculaire gauche (18) de manière à être comprimé par le déplacement de la membrane non élastique (10) de part et d'autre du plan de son support rigide périphérique (11), lesdits moyens d'isolement (19, 20) étant maintenus en place dans leurs espaces ventriculaires respectifs (17, 18) par fixation aux valves (22).

e) des moyens d'activation du dispositif moteur comportant au moins une source d'énergie qui génère une pression de fluide dans une chambre d'activation (34) délimitée par les deux membranes logeant entre elles le dispositif de soutien (5) de la membrane élastique (4), ladite pression d'activation provoquant une élongation de ladite membrane élastique (4) entraînant des déplacements de volume dans l'espace ventriculaire droit (17), le volume déplacé par la membrane (4) étant apte à être modifié en faisant varier la pression d'activation susdite, tandis que les déplacements de volume dans l'espace ventriculaire gauche (18) sont obtenus par flexion de la membrane nonélastique (10) sous l'effet de ladite pression, la différence d'action de la pression d'activation sur la membrane élastique (4), qu'elle soumet à une plus ou moins grande élongation en fonction de sa valeur, provoquant une activation asymétrique des ventricules droit et gauche et déterminant une variation correspondante du volume d'éjection du ventricule droit, et,

f) des moyens (36-41, 42-47) de régulation du débit sanguin du module en fonction des variations des résistances pulmonaire et systémique du porteur, ces moyens de régulation se composant de moyens (36-41) de réglage de la pression fluidique d'activation et du pourcentage de systole dans le cycle d'activation en fonction de la pression aortique ainsi que de moyens (42-47) de réglage de la durée du cycle d'activation en fonction de la pression de remplissage du ventricule gauche, l'ensemble de ces moyens de réglage (36-47) agissant en synergie.

2. Prothèse cardiaque totale selon la revendication 1, caractérisée en ce que la membrane (4) en matériau élastomère, qui travaille à l'élongation, présente une forme convexe et une surface, de l'ordre de 150 à 165 cm² environ.

3. Prothèse cardiaque totale selon la revendication 1, caractérisée en ce que ledit dispositif de soutien rigide (5) de la membrane élastique (4) qui travaille à l'élongation, est percé d'au moins une ouverture qui assure la communication de fluide à travers celui-ci.

4. Prothèse cardiaque totale selon l'une quelconque des revendications 1 à 3, caractérisée en ce que l'ensemble constitué par les deux membranes (4, 10) susdites et leurs dispositifs de soutien (5, 11), forme un ensemble étanche lui-même logé dans ledit boîtier (1, 2) en matériau étanche, auquel ledit ensemble est solidarisé par l'intermédiaire de logements (6, 7, 8, 9) appropriés ménagés dans ledit boîtier (1, 2) pour recevoir les extrémités des deux membranes et les extrémités de leurs supports respectifs.

5. Prothèse cardiaque totale selon l'une quelconque des revendications 1 à 4, caractérisée en ce que la distance entre les membranes (4, 10), qui délimitent avec les faces internes correspondantes du boîtier (1, 2), respectivement le ventricule gauche (18) et le ventricule droit (17), est croissante dans les trois dimensions spatiales, de la pointe vers la base de la prothèse, pour orienter les vecteurs vitesse du sang vers l'orifice valvulaire de sortie correspondant et éviter toutes stases.

6. Prothèse cardiaque totale selon l'une quelconque des revendications 1 à 5, caractérisée en ce que le volume de l'espace ventriculaire gauche (18) et le volume de l'espace ventriculaire droit (17) sont sensiblement égaux, tout en présentant une géométrie différente l'un de l'autre.

7. Prothèse cardiaque totale selon l'une quelconque des revendications 1 à 6, caractérisée en ce que chacune des valves (22) est constituée par un disque (33) en matériau hémocompatible approprié, dont le diamètre est sensiblement identique à celui de l'orifice valvulaire dans lequel il est monté, lequel disque est inséré entre une barrette d'articulation (31) et deux barrettes de butée (32) qui le bloquent en position ouverte ou fermée, en coopération avec ladite barrette d'articulation (31), créant en position ouverte une surface utile maximale et offrant une inertie minimale, et en ce que les inserts (21) sont conçus pour recevoir respectivement l'extrémité de fixation de la barrette d'articulation (31) et les extrémités de fixation des barrettes de butée (32) entre lesquelles est insérée la valve (22) susdite, et pour recevoir les extrémités des poches à sang (19, 20) qui sont fixées par pincement entre lesdits inserts et les moyens d'anastomose au porteur de la prothèse.

8. Prothèse cardiaque totale selon l'une quelconque des revendications 1 à 7, caractérisée en ce que les moyens d'activation du dispositif moteur (4, 5, 10, 11) sont constitués par un moteur électrique à piles implantable, dont l'énergie fournit les pressions d'alimentation requises au dispositif moteur susdit.

9. Prothèse cardiaque totale selon l'une quelconque des revendications 1 à 7, caractérisée en ce que les moyens d'activation du dispositif moteur sont constitués par un moteur électrique commandé par une source d'énergie nucléaire, qui fournissent les pressions d'alimentation requises au dispositif moteur (4, 5, 10, 11) susdit et sont tous deux implantables.

10. Prothèse cardiaque totale selon l'une quelconque des revendications 1 à 7, caractérisée en ce que les moyens d'activation du dispositif moteur sont constitués par une source d'énergie pneumatique extra-corporelle reliée à la prothèse cardiaque par un tube transcutané (16) de petit diamètre, réalisé en un matériau éliminant au maximum les risques d'infection transcutanée.

11. Prothèse cardiaque totale selon la revendication .10, caractérisée en ce que la source d'énergie pneumatique est constituée par deux pompes indépendantes agissant respectivement l'une sur la membrane élastique du ventricule droit, l'autre sur la membrane déformable du ventricule gauche.

12. Prothèse cardiaque totale selon la revendication 10 ou la revendication 11, caractérisée en ce que ledit tube transcutané (16) est recouvert de tissu réhabitable et équipé au contact des tissus, d'un disque en pyrolyte de carbone placé sous la peau qui empêche le cisaillement du tube sur la peau dans le sens axial et évite les infections.

13. Prothèse cardiaque totale selon l'une quelconque des revendications 1 à 12, caractérisée en ce que les moyens de régulation du débit sanguin (36-41, 42-47) fonctionnant par asservissement de la pression fluidique d'activation et du pourcentage du temps de systole dans le cycle d'activation à une valeur de référence de la pression aortique moyenne ainsi que par réglage de la durée du cycle d'activation en fonction de la pression de remplissage dans le ventricule gauche, limitent la fréquence à des valeurs comprises entre 80 et 110 cycles/minute.

14. Prothèse cardiaque totale selon la revendication 13, caractérisée en ce que lesdits moyens de régulation du débit sanguin comprennent, dans le cas où ils coopèrent avec des moyens d'activation constitués par une source d'énergie pneumatique, un premier moyen d'asservissement qui comporte un détendeur (48) de pression qui fait varier la pression d'activation entre 304 et 440 millibars, sous l'action d'un signal, un comparateur (38) qui émet ledit signal, après comparaison d'un signal correspondant à la pression effective du porteur de la prothèse avec un signal correspondant à une pression de référence, par exemple 133,326 millibars, si la pression effective diffère de la pression de référence, pour agir sur le détendeur (48) susdit dans le but d'augmenter ou de diminuer la pression d'activation exercée sur la membrane (4) qui délimite le ventricule droit (17) et d'augmenter ou diminuer le débit dans ledit ventricule droit (17), de façon correspondante ; et un deuxième moyen d'asservissement qui régule la période d'activation entre 750 et 530 millisecondes à l'aide d'un signal proportionnel émis consécutivement à l'établissement par un microprocesseur, associé à la capacitance (48) susdite, d'une relation proportionnelle entre la pression de remplissage du ventricule gauche (18) et la période du cycle d'activation, en fonction des variations de débit dans le ventricule droit (17) et de l'augmentation résultante de la pression de remplissage du ventricule gauche (18), qui est représentée par un signal de pression représentatif de la pression de remplissage du ventricule gauche (18), émis par ladite capacitance (48) associée à l'activateur pneumatique.

15. Prothèse cardiaque totale selon l'une quelconque des revendications 13 et 14, caractérisée en ce que lesdits moyens de régulation du débit cardiaque comprennent un troisième moyen d'asservissement qui détecte la pression de remplissage du ventricule droit (17) et est associé à un comparateur qui compare cette pression avec une pression de référence, le résultat de cette comparaison commandant la valeur de la pression de référence déterminée du moyen d'asservissement à la pression aortique.

16. Prothèse cardiaque totale selon la revendication 15, caractérisée en ce que le troisième moyen d'asservissement, qui mesure les valeurs de pression de remplissage du ventricule droit (17), les enregistre pendant une durée prédéterminée et transmet leur valeur moyenne à un comparateur qui les compare à la valeur de la pression de référence prédéterminée et commande le changement de la valeur prédéterminée de la pression aortique de référence.

17. Prothèse cardiaque totale selon l'une quelconque des revendications 15 et 16, caractérisée en ce que le troisième moyen d'asservissement comporte un capteur (50) de pression de remplissage du ventricule droit (18), ce capteur (50) envoyant son signal de sortie dans un intégrateur ou enregistreur dont le signal de sortie est appliqué à un comparateur (51) pour être comparé à un signal prédéterminé de référence engendré par un générateur (52), ce comparateur (51) commandant le générateur (52) du signal de référence de la pression aortique du comparateur (38) du moyen d'asservissement à la pression aortique, ou premier moyen d'asservissement.

18. Dispositif de régulation du débit sanguin dans une prothèse cardiaque totale selon l'une quelconque des revendications 1 à 17, du type comportant deux moyens d'asservissement, caractérisé en ce que le premier moyen d'asservissement établit une relation directe entre la pression aortique moyenne d'une part, et d'autre part la pression systolique d'activation et le pourcentage du temps de systole dans le cycle, tandis que le deuxième moyen d'asservissement établit une relation entre la pression moyenne de remplissage du ventricule gauche et la fréquence cardiaque, une relation indirecte s'établissant ainsi entre le premier et le deuxième moyen d'asservissement qui agissent en synergie, en raison du fait que le premier moyen d'asservissement modifie la pression moyenne de remplissage du ventricule gauche en faisant varier le volume systolique droit et le deuxième moyen d'asservissement fait varier la fréquence cardiaque lorsque varie la pression moyenne de remplissage du ventricule gauche.

19. Dispositif de régulation du débit sanguin selon la revendication 18, caractérisé en ce qu'il comprend, en outre, un troisième moyen d'asservissement qui commande des variations de la pression aortique en fonction des variations de volume du sang circulant, lesquelles se traduisent par une variation de la pression de remplissage du ventricule droit (18), dont la mesure et la comparaison par un comparateur (51) avec une pression moyenne prédéterminée commandent la variation de la pression aortique initiale.

**Claims**

1. A complete cardiac prosthesis comprising :
   a) a rigid case (1, 2) made from a sealed and non toxic material compatible with respect to the surrounding tissues, said case defining a biventricular one piece cardiac module having a form and a volume making possible the implantation thereof in the pericardial space, exactly in the position and in place of the natural heart,
   b) a drive device housing the case (1, 2) and having two flexible membranes (4, 10) one (4) of which, made from elastomer, defines with the corresponding internal face of the case (1, 2) situated opposite, a space which forms the right ventricle (17), whereas the other (10), non elastic and having an area substantially less than that of the first membrane (4), defines with a corresponding internal face of the case (1, 2) situated opposite, a left ventricular space (18), the elastic membrane (4) taking on the shape and being applied to the convex surface of a rigid support device (5) in its non operating condition, whereas the non elastic membrane (10) is fixed peripherally to a rigid support (11),

c) valves (22) mounted in valvular orifices (12, 13, 14, 15) of the case by means of inserts (21) intended for an anastomosis with the vessels of the circulatory system, said inserts being designed so as to confer on each valve (22) an inner diameter substantially identical to that of the valvular orifice in which it is mounted,

d) means for isolating the blood with respect to the case and the drive device, said means being formed by blood pockets or bags made from isolating hemo-compatible materials, a first blood bag (19) being housed in the right ventricular space (17) so as to be compressed by elongation of the elastic membrane (4) and a second blood bag (20) being housed in the left ventricular space (18) so as to be compressed by movement of the non elastic membrane (10) on each side of the plane of its peripheral rigid support (11), said isolating means (19, 20) being held in position in their respective ventricular spaces (17, 18) by fixing to the valves (22).

e) means for activating the drive device including at least one energy source which generates a fluid pressure in an activation chamber (24) defined by the two membranes housing therebetween the device (5) supporting the elastic membrane (4) said activation pressure causing an elongation of said elastic membrane (4) resulting in volume displacements in the right ventricular space (17), the volume displaced by the membrane (4) being adapted to be modified by varying said activation pressure, whereas the volume displacements in the left ventricular space (18) are obtained by flexion of the non elastic membrane (10) under the effect of said pressure, the difference of action of the activation pressure on the elastic membrane (4), which it subjects to a more or less great elongation depending on this value, causing an asymmetric activation of the right and left ventricles and determining a corresponding variation of the ejection volume of the right ventricle, and

f) means (36-41, 42-47) for regulating the blood flow of the module as a function of the variations of the pulmonary and systemic resistances of the bearer, these regulation means comprising means (36-41) for adjusting the activation fluid pressure and the systole percentage in the activation cycle as a function of the aortic pressure as well as means (42-47) for adjusting the duration of the activation cycle as a function of the filling pressure of the left ventricle, the whole of these adjustment means (36-47) acting together.

2. Complete cardiac prosthesis according to claim 1, characterized in that the elastomer material membrane (4), which works under elongation, has a convex form and an area of the order of about 150 to 165 cm².

3. Complete cardiac prosthesis according to claim 1, characterized in that said rigid device (5) supporting the elastic membrane (4) which works under elongation, is pierced with a least one opening which ensures fluid communication therethrough.

4. Complete cardiac prosthesis according to any one of claims 1 to 3, characterized in that the whole formed by said two membranes (4, 10) and their support devices (5, 11), form a sealed assembly self housed in said sealed material case (1, 2), to which said assembly is made fast by means of appropriate housings (6, 7, 8, 9) formed in said case (1, 2) for receiving the ends of the two membranes and the ends of their respective supports.

5. Complete cardiac prosthesis according to any one of claims 1 to 4, characterized in that the distance between the membranes (4, 10), which define, with the corresponding internal faces of the case (1, 2), respectively the left ventricle (18) and the right ventricle (17), is increasing in the three spatial dimensions, from the tip to the base of the prosthesis, for orienting the speed vectors of the blood towards the corresponding valvular outlet orifice and for avoiding any stases.

6. Complete cardiac prosthesis according to any one of claims 1 to 5, characterized in that the volume of the left ventricular space (18) and the volume of the right ventricular space (17) are substantially equal, while having a different geometry with respect to each other.

7. Complete cardiac prosthesis according to any one of claims 1 to 6, characterized in that each of the valves (22) is formed by a disk (33) made from an appropriate hemo-compatible material, whose diameter is substantially identical to that of the valvular orifice in which it is mounted, which disk is inserted between an articulation bar (31) and two abutment bars (32) which block it in the opened or closed position, in cooperation with said articulation bar (31), creating in the open position a maximum useful area and offering minimum inertia, and in that the inserts (21) are designed for receiving respectively the fixing end of the articulation bar (31) and the fixing ends of the abutment bars (32) between which said valve (22) is inserted, and for receiving the ends of the blood pockets (19, 20) which are fixed by nipping between said inserts and the means for anastomosis to the bearer of the prosthesis.

8. Complete cardiac prosthesis according to any one of claims 1 to 7, characterized in that the means for activating the drive device (4, 5, 10, 11) are formed by an implantable battery driven electric motor, whose energy provides the supply pressures required to said drive device.

9. Complete cardiac prosthesis according to any one of claims 1 to 7, characterized in that the means for activating the drive device are formed by an electric motor controlled by a nuclear energy source, which deliver the required supply pressures to said drive device (4, 5, 10, 11) and are both implantable.

10. Complete cardiac prosthesis according to any one of claims 1 to 7, characterized in that the means for activating the drive device are formed by an extracorporeal pneumatic energy source connected to the cardiac prosthesis by a transcutaneous tube (16) of small diameter, made from a material removing as much as possible the risks of transcutaneous infection.

11. Complete cardiac prosthesis according to claim 10, characterized in that the pneumatic energy source is formed by two independent pumps acting respectively one on the elastic membrane of the right ventricle, the other on the deformable membrane of the left ventricle.

12. Complete cardiac prosthesis according to claim 10 or claim 11, characterized in that said transcutaneous tube (16) is coated with rehabitable tissue and equipped, in contact with the tissues, with a carbon pyrolyte disk placed under the skin which prevents shearing of the tube on the skin in the axial direction and prevents infections.

13. Complete cardiac prosthesis according to any one of claims 1 to 12, characterized in that the means for regulating the blood flow rate (36-41, 42-47) operating by slaving of the fluid activation pressure and of the systol time percentage in the activation cycle to a reference value of the mean aortic pressure as well as by adjustment of the duration of the activation cycle as a function of the filling pressure in the left ventricle, limit the frequency to the values between 80 and 110 cycles/min.

14. Complete cardiac prosthesis according to claim 13, characterized in that said means for regulating the blood flow rate comprise, in the case where they cooperate with activation means formed by a pneumatic energy source, a first slaving means which comprise a pressure reducer (48) which causes the activation pressure to vary between 304 and 440 millibars, under the action of the signal, a comparator (38) which delivers said signal after comparison of a signal corresponding to the effective pressure of the bearer of the prosthesis with a signal corresponding to a reference pressure, for example 133.326 millibars, if the effective pressure differs from the reference pressure, for acting on said pressure reducer (48) for increasing or reducing the activation pressure exerted on the membrane (4) which defines the right ventricle (17) and for increasing or reducing the flow rate in said right ventricle (17), correspondingly ; and a second slaving means which regulates the activation period between 750 and 530 milliseconds by means of a proportional signal delivered following the establishment by a microprocessor, associated with said capacitance (48), of a proportional regulation between the filling pressure of the left ventricle (18) and the period of the activation cycle, as a function of the flow rate variations in the right ventricle (17) and the resulting increase of the filling pressure of the left ventricle (18), which is represented by a pressure signal representative of the filling pressure of the left ventricle (18), delivered by said capacitance (48) associated with the pneumatic activator.

15. Complete cardiac prosthesis according to any one of claims 13 and 14, characterized in that said means for regulating the cardiac flow rate comprise a third slaving means which detects the filling pressure of the right ventricle (17) and is associated with a comparator which compares this pressure with a reference pressure, the result of this comparison controlling the value of the determined reference pressure of the means for slaving to the aortic pressure.

16. Complete cardiac prosthesis according to claim 15, characterized in that the third slaving means, which measures the filling pressure values of the right ventricle (17), stores them for a predetermined time and transmits their mean value to a comparator which compares them with a value of the predetermined reference pressure and controls the change of the predetermined value of the reference aortic pressure.

17. Complete cardiac prosthesis according to any one of claims 15 and 16, characterized in that the third slaving means comprise a sensor (50) detecting the filling pressure of the right ventricle (18), this sensor (50) sending its output signal to an integrator or recorder whose output signal is applied to a comparator (51) for comparison with a predetermined reference signal generated by a generator (52), this comparator (51) controlling the generator (52) generating the reference signal of the aortic pressure of the comparator (38) of the means for slaving to the aortic pressure, or first.

18. Device for regulating the blood flow rate in a complete cardiac prosthesis according to any one of claims 1 to 17, of the type having two slaving means, characterized in that the first slaving means establishes a direct relationship between the mean aortic pressure on the one hand and, on the other hand, the systolic activation pressure and the percentage of systole time in the cycle, whereas the second slaving means establishes a relationship between the mean filling pressure of the left ventricle and the cardiac frequency, an indirect relationship thus being established between the first and second slaving means which act together, because of the fact that the first slaving means modifies the mean filling pressure of the left ventricle by varying the right systolic volume and the second slaving means causes the cardiac frequency to vary when the mean filling pressure of the left ventricle varies.

19. Device for regulating the blood flow rate according to claim 18, characterized in that it further comprises a third slaving means which controls variations of the aortic pressure as a function of the volume variations of the circulating blood which result in a variation of the filling pressure of the right ventricle (18), the measurement and comparison of which by a comparator (51) with a mean predetermined pressure control the variation of the initial aortic pressure.

**Patentansprüche**

1. Vollständige Herzprothese, umfassend :
  a) ein starres Gehäuse (1, 2) aus einem dichten Werkstoff, der gegenüber den umgebenden Geweben verträglich und nichttoxisch ist, wobei das Gehäuse ein Zwei-Kammer-Herzmodul aus einem Block begrenzt, das eine Form und ein Volumen aufweist, die bzw. das seine Implantation in den Perikardraum genau an Ort und Stelle des natürlichen Herzens ermöglicht,

b) eine im Gehäuse (1, 2) angeordnete Antriebseinrichtung, umfassend zwei biegsame Membranen (4, 10), von denen eine (4) aus einem Elastomer mit der entsprechenden gegenüberliegenden Innenseite des Gehäuses (1, 2) einen Raum begrenzt, der die rechte Herzkammer (17) ausmacht, während die andere (10), nichtelastisch und mit deutlich kleinerer Oberfläche als die erste Membran (4), mit der entsprechenden gegenüberliegenden Innenseite des Gehäuses (1, 2) einen linken Herzkammerraum (18) begrenzt, wobei die elastische Membran (4) in ihrem Ruhezustand die Form der konvexen Oberfläche einer starren Stützeinrichtung (5) annimmt und sich an diese anlegt, während die nichtelastische Membran (10) peripherisch auf einem starren Träger (11) befestigt ist,

c) Ventile (22), montiert in Ventilöffnungen (12, 13, 14, 15) des Gehäuses mit Hilfe von Einsatzteilen (21), bestimmt zur Anastomose mit den Gefäßen des Kreislaufnetzes, wobei die Einsatzteile so beschaffen sind, daß sie jedem Ventil (22) einen Innendurchmesser verleihen, der im wesentlichen identisch ist mit demjenigen der Ventilöffnung, in der das Ventil montiert ist,

d) Mittel zum Isolieren des Blutes gegenüber dem Gehäuse und der Antriebseinrichtung, bestehend aus Bluttaschen oder Blutbeuteln aus isolierenden blutverträglichen Werkstoffen, wobei ein erster Blutbeutel (19) im rechten Herzkammerraum (17) so angeordnet ist, daß er durch die Dehnung der elastischen Membran (4) zusammengedrückt wird, und ein zweiter Blutbeutel (20) im linken Herzkammerraum (18) so angeordnet ist daß er durch die Verschiebung der nichtelastischen Membran (10) zu beiden Seiten der Ebene ihres peripheren starren Trägers (11) zusammengedrückt wird, und wobei die Isoliermittel (19, 20) in ihren jeweiligen Herzkammerräumen (17, 18) durch Fixierung an die Ventile (22) an Ort und Stelle gehalten werden,

e) Mittel zum Aktivieren der Antriebseinrichtung, umfassend mindestens eine Energiequelle, die einen Flüssigkeitsdruck in einer Aktivierungskammer (34) erzeugt, die begrenzt wird von den beiden Membranen, zwischen denen sich die Stützvorrichtung (5) der elastischen Membran (4) befindet, wobei der Aktivierungsdruck eine Dehnung der elastischen Membran (4) bewirkt, die zu Volumenverschiebungen bzw. -verdrängungen im rechten Herzkammerraum (17) führt, wobei das durch die Membran (4) verdrängte Volumen modifiziert werden kann, indem man den oben genannten Aktivierungsdruck variiert, während die Volumenverschiebungen bzw. -verdrängungen im linken Herzkammerraum (18) mittels Durchfederung der nichtelastischen Membran (10) unter Einwirkung des genannten Druckes erhalten werden, wobei die unterschiedliche Einwirkung des Aktivierungsdrucks auf die elastische Membran (4), die von diesem je nach seiner Stärke mehr oder weniger stark gedehnt wird, eine asymmetrische Aktivierung der rechten und der linken Herzkammern hervorruft und eine entsprechende Schwankung des Ausstoßvolumens der rechten Herzkammer bestimmt, und

f) Mittel (36-41, 42-47) zum Regeln des Minutenvolumens bzw. Blutdurchflusses des Moduls in Abhängigkeit von den Schwankungen des Lungenwiderstands und des systemischen Widerstands des Trägers, wobei diese Mittel zum Regeln sich zusammensetzen aus Mitteln (36-41) zum Regeln des Flüssigkeits-Aktivierungsdrucks und des prozentualen Anteils der Systole im Aktivierungszyklus in Abhängigkeit vom Aorta-Druck, sowie aus Mitteln (42-47) zum Regeln der Dauer des Aktivierungszyklus in Abhängigkeit von dem Fülldruck der linken Herzkammer, wobei diese Mittel zum Regeln (36-47) insgesamt in Synergie wirken.

2. Vollständige Herzprothese nach Anspruch 1, dadurch gekennzeichnet, daß die Membran (4) aus elastomerem Werkstoff, die mit Dehnung arbeitet, eine konvexe Form und eine Oberfläche in der Größenordnung von etwa 150 bis 165 cm² aufweist.

3. Vollständige Herzprothese nach Anspruch 1, dadurch gekennzeichnet, daß die starre Stützeinrichtung (5) der elastischen Membran (4), die mit Dehnung arbeitet, mindestens eine Öffnung aufweist, die die Flüssigkeitskommunikation durch sie hindurch sicherstellt.

4. Vollständige Herzprothese nach einem der Ansprüche 1 bis 3, dadurch gekennzeichnet, daß die aus den beiden Membranen (4, 10) und ihren Stützeinrichtungen (5, 11) bestehende Gesamtheit ein dichtes Ganzes bildet, das in dem Gehäuse (1, 2) angeordnet und mit diesem durch geeignete Aufnahmen (6, 7, 8, 9) aus dichtem Werkstoff fest verbunden ist, die in dem Gehäuse (1, 2) vorgesehen sind, um die Enden der beiden Membranen und die Enden ihrer jeweiligen Träger aufzunehmen.

5. Vollständige Herzprothese nach einem der Ansprüche 1 bis 4, dadurch gekennzeichnet, daß der Abstand zwischen den Membranen (4, 10), die mit den entsprechenden Innenseiten des Gehäuses (1, 2) die linke Herzkammer (18) und die rechte Herzkammer (17) begrenzen, in den drei Dimensionen des Raumes zunimmt von der Spitze zur Basis der Prothese, um die Blutgeschwindigkeits-Vektoren zur entsprechenden Ausgangs-Ventilöffnung zu richten und jegliche Stauungen zu vermeiden.

6. Vollständige Herzprothese nach einem der Ansprüche 1 bis 5, dadurch gekennzeichnet, daß das Volumen des linken Herzkammerraums (18) und das Volumen des rechten Herzkammerraums (17) im wesentlichen gleich sind, während sie eine voneinander verschiedene Geometrie aufweisen.

7. Vollständige Herzprothese nach einem der Ansprüche 1 bis 6, dadurch gekennzeichnet, daß jedes Ventil (22) aus einer Scheibe (33) aus geeignetem blutverträglichen Werkstoff besteht, deren Durchmesser im wesentlichen identisch ist mit demjenigen der Ventilöffnung, in der sie montiert ist, wobei die Scheibe zwischen einem Gelenkstab (31) und zwei Stoppstäben (32) eingelassen ist, die sie in Auf- oder Zu-Stellung blockieren in Zusammenwirkung· mit dem Gelenkstab (31), wobei bei Auf-Stellung eine maximale Nutzfläche erzeugt und minimale Trägheit geboten wird und die Einsätze (21) ausgebildet sind, um jeweils das Befestigungsende des Gelenkstabs (31) und die Befestigungsenden der Stoppstäbe (32), zwischen die das Ventil (22) eingesetzt ist, aufzunehmen und um die Enden der Blutbeutel (19, 20) aufzunehmen, die durch Klemmen zwischen den Einsätzen und den Anastomosemitteln an dem Träger der Prothese fixiert sind.

8. Vollständige Herzprothese nach einem der Ansprüche 1 bis 7, dadurch gekennzeichnet, daß die Mittel zum Aktivieren der Antriebseinrichtung (4, 5, 10, 11) aus einem implantierbaren Elektromotor mit Batterien bestehen, dessen Energie die benötigten Primärdrücke der Antriebseinrichtung liefert.

9. Vollständige Herzprothese nach einem der Ansprüche 1 bis 7, dadurch gekennzeichnet, daß die Aktivierungsmittel der Antriebseinrichtung aus einem Elektromotor bestehen, der von einer Kernenergiequelle gesteuert wird, die die benötigten Primärdrücke an die Antriebseinrichtung (4, 5, 10, 11) liefern, und beide implantierbar sind.

10. Vollständige Herzprothese nach einem der Ansprüche 1 bis 7, dadurch gekennzeichnet, daß die Aktivierungsmittel der Antriebseinrichtung aus einer extrakorporalen pneumatischen Energiequelle bestehen, die mit der Herzprothese durch einen transkutanen Schlauch (16) von kleinem Durchmesser, ausgeführt aus einem Werkstoff, der so weit wie möglich die Risiken einer transkutanen Infektion eliminiert, verbunden ist.

11. Vollständige Herzprothese nach Anspruch 10, dadurch gekennzeichnet, daß die pneumatische Energiequelle aus zwei unabhängigen Pumpen besteht, von denen eine auf die elastische Membran der rechten Herzkammer und die andere auf die deformierbare Membran der linken Herzkammer wirkt.

12. Vollständige Herzprothese nach Anspruch 10 oder Anspruch 11, dadurch gekennzeichnet, daß der transkutane Schlauch (16) mit einem rehabitablen Gewebe überzogen und bei Berührung der Gewebe mit einer Scheibe aus Kohlenstoffpyrolyt ausgestattet ist, die unter der Haut angeordnet ist und das Scheren des Schlauches auf der Haut in axialer Richtung verhindert und die Infektionen vermeidet.

13. Vollständige Herzprothese nach einem der Ansprüche 1 bis 12, dadurch gekennzeichnet, daß die Mittel zum Regulieren des Blutdurchflusses (36-41, 42-47), die durch (Ein)Regeln des Flüssigkeits-Aktivierungsdrucks und der prozentualen Zeit der Systole im Aktivierungszyklus auf einen Bezugswert des mittleren Aortadrucks sowie durch Regeln der Dauer des Aktivierungszyklus in Abhängigkeit vom Fülldruck in der linken Herzkammer arbeiten, die Frequenz auf Werte zwischen 80 und 110 Schläge/Minute begrenzen.

14. Vollständige Herzprothese nach Anspruch 13, dadurch gekennzeichnet, daß die Mittel zum Regulieren des Blutdurchflusses dann, wenn sie mit Aktivierungsmitteln, bestehend aus einer pneumatischen Energiequelle, zusammenwirken, ein erstes Mittel zum (Ein)Regulieren umfassen, das besteht aus einem Druckreduzierventil (48), das den Aktivierungsdruck zwischen 304 und 440 mbar variiert unter der Einwirkung eines Signals, aus einem Komparator (38), der das Signal ausgibt nach Vergleich eines Signals das dem Effektivdruck des Trägers der Prothese entspricht, mit einem Signal, das dem Bezugsdruck, beispielsweise 133,326 mbar, entspricht, wenn der Effektivdruck vom Bezugsdruck verschieden ist, um auf das Druckreduzierventil (48) einzuwirken, um den Aktivierungsdruck zu erhöhen oder zu verringern, der auf die Membran (4) ausgeübt wird, die die rechte Herzkammer (17) begrenzt, und um den Blutdurchfluß in dieser rechten Herzkammer (17) in entsprechender Weise zu erhöhen oder zu verringern ; und ein zweites Mittel zum (Ein)Regulieren, das die Aktivierungsperiode zwischen 750 und 530 ms reguliert mit Hilfe eines Proportionalsignals, das ausgegeben wird, nachdem mit einem Mikroprozessor, der mit der Kapazitanz bzw. dem Druckventil (48) kombiniert ist, eine Proportionalrelation hergestellt worden ist zwischen dem Fülldruck der linken Herzkammer (18) und· der Periode des Aktivierungszyklus in Abhängigkeit von den Schwankungen des Blutdurchflusses in der rechten Herzkammer (17) und der sich daraus ergebenden Erhöhung des Fülldrucks der linken Herzkammer (18), die wiedergegeben ist durch ein Drucksignal repräsentativ für den Fülldruck der rechten Herzkammer (18), ausgegeben von der Kapazitanz (48), die mit dem pneumatischen Aktivator kombiniert ist.

15. Vollständige Herzprothese nach einem der Ansprüche 13 und 14, dadurch gekennzeichnet, daß die Mittel zum Regulieren der Herzleistung ein drittes Mittel zum (Ein)Regulieren umfassen, das den Fülldruck der rechten Herzkammer (17) nachweist und verbunden ist mit einem Komparator, der diesen Druck mit einem Bezugsdruck vergleicht, wobei das Ergebnis dieses Vergleichs den Wert des bestimmten Bezugsdrucks des Mittels zum Einregulieren an den Aortadruck steuert.

16. Vollständige Herzprothese nach Anspruch 15, dadurch gekennzeichnet, daß das dritte Mittel zum (Ein)Regulieren, das die Werte des Füldrucks der rechten Herzkammer (17) mißt, sie während einer vorbestimmten Zeitdauer registriert und ihren Mittelwert an einen Komparator weitergibt, der sie mit dem vorbestimmten Bezugsdruck vergleicht und die Änderung des vorbestimmten Wertes des Aorta-Bezugsdrucks steuert.

17. Vollständige Herzprothese nach einem der Ansprüche 15 und 16, dadurch gekennzeichnet, daß das dritte Mittel zum (Ein)Regulieren einen Aufnehmer bzw. eine Meßzelle (50) für den Fülldruck der rechten Herzkammer (18) umfaßt, dieser Aufnehmer (50) bzw. diese Meßzelle sein bzw. ihr Ausgangssignal in einen Integrator oder Schreiber schickt, dessen Ausgangssignal an einen Komparator (51) angelegt wird, um mit einem vorbestimmten Bezugssignal verglichen zu werden, das von einem Generator (52) erzeugt wird, der Komparator (51) den Generator (52) des Bezugssignals des Aortadrucks des Komparators (38) des Mittels zum (Ein)Regulieren des Aortadrucks oder ersten Mittels zum (Ein)Regulieren steuert.

18. Vorrichtung zur Regulierung des Blutdurchflusses in einer vollständigen Herzprothese nach einem der Ansprüche 1 bis 17 vom Typ, umfassend zwei Mittel zum (Ein)Regulieren, dadurch gekennzeichnet, daß das erste Mittel zum (Ein)Regulieren eine direkte Beziehung herstellt zwischen dem mittleren Aortadruck einerseits und dem systolischen Aktivierungsdruck und der prozentualen Zeit der Systole im Zyklus andererseits, während das zweite Mittel zum (Ein) Regulieren eine Beziehung herstellt zwischen dem mittleren Fülldruck der linken Herzkammer und der Herzfrequenz, wodurch eine indirekte Beziehung hergestellt wird zwischen dem ersten und dem zweiten Mittel zum (Ein)Regulieren, die in Synergie wirken aufgrund der Tatsache, daß das erste Mittel zum (Ein)Regulieren den mittleren Fülldruck der linken Herzkammer modifiziert durch Ändern des rechten systolischen Volumens und das zweite Mittel zum (Ein)Regulieren die Herzfrequenz ändert, wenn der mittlere Fülldruck der linken Herzkammer sich ändert.

19. Vorrichtung zum Regulieren des Blutdurchflusses nach Anspruch 18, dadurch gekennzeichnet, daß sie zusätzlich ein drittes Mittel zum (Ein)Regulieren umfaßt, das die Änderungen des Aortadrucks steuert in Abhängigkeit von den Änderungen des umlaufenden Blutvolumens, die sich in einer Änderung des Fülldrucks der rechten Herzkammer (18) äußern, dessen Messung und Vergleich durch einen Komparator (51) mit einem vorgegebenen mittleren Druck die Änderung bzw. Schwankung des Anfangs-Aortadrucks steuern.

0 014 130

Fig. 1

Fig. 2

0 014 130

Fig. 3

Fig. 4

Fig. 8

Fig. 5

Fig. 6

42

43

47

44

46

45

31

32

32

33

22

28

27

26

29

23

21

25

24

30

# Fig.7

# Fig. 9